(19) Europäisches Patentamt
European Patent Office
Office européen des brevets

(11) **EP 3 134 610 B1**

(12) **EUROPEAN PATENT SPECIFICATION**

(45) Date of publication and mention of the grant of the patent:
**20.11.2019 Bulletin 2019/47**

(51) Int Cl.:
*G01N 11/00* (2006.01)       *G01N 9/36* (2006.01)
*E21B 43/12* (2006.01)       *E21B 47/00* (2012.01)
*G01N 33/28* (2006.01)

(21) Application number: **14892867.4**

(22) Date of filing: **23.05.2014**

(86) International application number:
**PCT/US2014/039410**

(87) International publication number:
**WO 2015/178934 (26.11.2015 Gazette 2015/47)**

(54) **ROBUST VISCOSITY ESTIMATION METHODS AND SYSTEMS**

VERFAHREN UND SYSTEME ZUR ROBUSTEN VISKOSITÄTSSCHÄTZUNG

PROCÉDÉS ET SYSTÈMES D'ESTIMATION ROBUSTE DE VISCOSITÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**01.03.2017 Bulletin 2017/09**

(73) Proprietor: **Landmark Graphics Corporation Houston, Texas 77042 (US)**

(72) Inventors:
 • **LIU, Zhengchun**
  **Sugar Land, TX 77479 (US)**
 • **SAMUEL, Robello**
  **Cypress, TX 77433 (US)**
 • **GONZALES, Adolfo**
  **Houston, TX 77095 (US)**
 • **KANG, Yongfeng**
  **Katy, TX 77494 (US)**

(74) Representative: **King, Lawrence et al
A.A. Thornton & Co.
10 Old Bailey
London EC4M 7NG (GB)**

(56) References cited:
 EP-B1- 2 115 486       WO-A1-2011/123093
 FR-A1- 2 602 054       US-A- 3 720 096
 US-A1- 2002 066 856    US-A1- 2013 025 359

 US-B2- 7 511 488

 • HANS PETTER ROENNINGSEN: "Prediction of viscosity and surface tension of North Sea petroleum fluids by using the average molecular weight", ENERGY & FUELS., vol. 7, no. 5, 1 September 1993 (1993-09-01), pages 565-573, XP055427969, WASHINGTON, DC, US. ISSN: 0887-0624, DOI: 10.1021/ef00041a001
 • OZDOGAN S ET AL: "Correlations towards prediction of petroleum fraction viscosities: an empirical approach", FUEL, IPC SCIENCE AND TECHNOLOGY PRESS, GUILDFORD, GB, vol. 80, no. 3, 1 February 2001 (2001-02-01), pages 447-449, XP027221679, ISSN: 0016-2361
 • WAKABAYASHI T ED - BORREGO ANGELES G ET AL: "Viscosity correlation with specific gravity and molecular weight of crude oil fractions", F, IPC SCIENCE AND TECHNOLOGY PRESS, GUILDFORD, GB, vol. 76, no. 11, 1 September 1997 (1997-09-01), pages 1049-1056, XP004286923, ISSN: 0016-2361, DOI: 10.1016/S0016-2361(97)00090-2
 • A I MYTAREVA ET AL: "Emperical relationships between crude-oil characteristics", CHEMISTRY AND TECHNOLOGY OF FUELS AND OILS, vol. 48, no. 5, 30 November 2012 (2012-11-30), pages 403-408, XP009501791, ISSN: 0009-3092, DOI: 10.1007/s10553-012-0387-3

• **AL-BALUSHI MAJDA ET AL: "Parametric study to develop an empirical correlation for undersaturated crude oil viscosity based on the minimum measured input parameters", FUEL, IPC SCIENCE AND TECHNOLOGY PRESS, GUILDFORD, GB, vol. 119, 2 December 2013 (2013-12-02), pages 111-119, XP028670848, ISSN: 0016-2361, DOI: 10.1016/J.FUEL.2013.11.044**

**Description**

BACKGROUND

It is widely accepted in the oil and gas industry that viscosity is the single most important transport property for subsurface simulations, well design, and pipeline and process simulations. As crude oil viscosity varies with temperature, pressure, and composition, there are many existing models for estimating crude oil viscosity. Unfortunately, some of these models require difficult-to-obtain measurements. Another issue is that many models are not reliable over the desired range of temperature, pressure, and compositions. In particular, high-pressure high-temperature (HPHT) conditions are problematic.

In H.P. Roenningsen "Prediction of viscosity and surface tension of North Sea petroleum fluids by using the average molecular weight", ENERGY&FUELS, Vol. 7, No. 5 (September/October 1993) a method for calculating crude oil viscosity from molecular weight is disclosed. In US 3 720 096 a method for calculating molecular weight from API gravity is disclosed.

SUMMARY OF THE INVENTION

According to the present invention, there is provided a computer-implemented method according to claim 1 and a system according to claim 8.

BRIEF DESCRIPTION OF THE DRAWINGS

Accordingly, there are disclosed in the drawings and the following description robust viscosity estimation methods and systems. In the drawings:

FIG. 1 shows an illustrative hydrocarbon production system.
FIG. 2A shows an illustrative logging while drilling (LWD) environment.
FIG. 2B shows an illustrative wireline logging environment.
FIG. 2C shows an illustrative permanent well environment.
FIG. 3 shows an illustrative chart of crude oil viscosity changes with temperature and pressure.
FIG. 4 shows an illustrative chart correlating effective molecular weight with API gravity.
FIG. 5A shows an illustrative chart showing accuracies of the disclosed viscosity model.
FIG. 5B shows an illustrative chart showing accuracies of a traditional Beal-Standing viscosity model.
FIG. 6A shows an illustrative chart showing absolute relative errors of the disclosed viscosity model.
FIG. 6B shows an illustrative chart showing absolute relative errors of a traditional Beal-Standing viscosity model.
FIG. 7A shows an illustrative chart showing accuracies of the disclosed viscosity model for dead oil with K-factor.
FIG. 7B shows an illustrative chart showing accuracies of the disclosed viscosity model for dead oil without K-factor.
FIG. 8A shows an illustrative chart showing accuracies of the disclosed viscosity model for live oil.
FIG. 8B shows an illustrative chart showing accuracies of a Vasquez-Beggs viscosity model for live oil.
FIG. 9A shows an illustrative chart showing deviation of existing viscosity models at high-pressure high-temperature (HPHT) conditions.
FIG. 9B shows an illustrative chart showing viscosity prediction results of various models at HPHT conditions.
FIG. 10A-10C show illustrative charts showing predicted viscosity values at different pressures and temperatures.
FIG. 11 shows an illustrative workflow for employing the disclosed viscosity model.
FIG. 12 shows an illustrative method for estimating viscosity.

It should be understood, however, that the specific embodiments given in the drawings and detailed description do not limit the disclosure. On the contrary, they provide the foundation for one of ordinary skill to discern the alternative forms, equivalents, and modifications that are encompassed together with one or more of the given embodiments in the scope of the appended claims.

DETAILED DESCRIPTION

Disclosed herein are methods and systems for robust estimation of crude oil viscosity. In the method according to the present invention, API gravity as a relative density of crude oil is first obtained. The relative density value may be obtained, for example, from previous and/or ongoing measurements of crude oil at any point in a pre-production environment, production system, or post-production system. Using the obtained relative density value, an effective molecular weight of the crude oil is determined. For example, a curve that correlates API gravity with effective molecular weight may be used to determine the effective molecular weight. Further, the effective molecular weight may be modified by applying a Watson characterization adjustment, a gas content adjustment, and/or a relative pressure adjustment. For example, an effective molecular weight determined using the relative density value and a Watson characterization factor (if available) is suitable for calculating a deal oil viscosity. Meanwhile, an effective molecular weight (with or without Watson characterization adjustment) with gas content adjustment is suitable for calculating a saturated oil viscosity. Further, an effective molecular weight (with or without Watson characterization adjustment) with a gas content adjustment and a relative pressure adjustment is suitable for calculating an under-saturated oil viscosity. The disclosed viscosity model calculates crude oil viscosity as a function of the effective molecular weight (or modified variations), temperature, and pressure. In some cases, crude oil viscosity is calculated by scaling a crude oil viscosity value corresponding to an effective molecular weight with gas content adjustment for a gas/oil ratio at bubble point pressure. The calculated crude oil viscosity may be used in fluid flow simulations used for pre-production, production, and/or post-production operations. For example, well completion operations, production settings, and/or enhanced oil recovery settings may be based at least in part on crude oil viscosities calculated using the disclosed viscosity model.

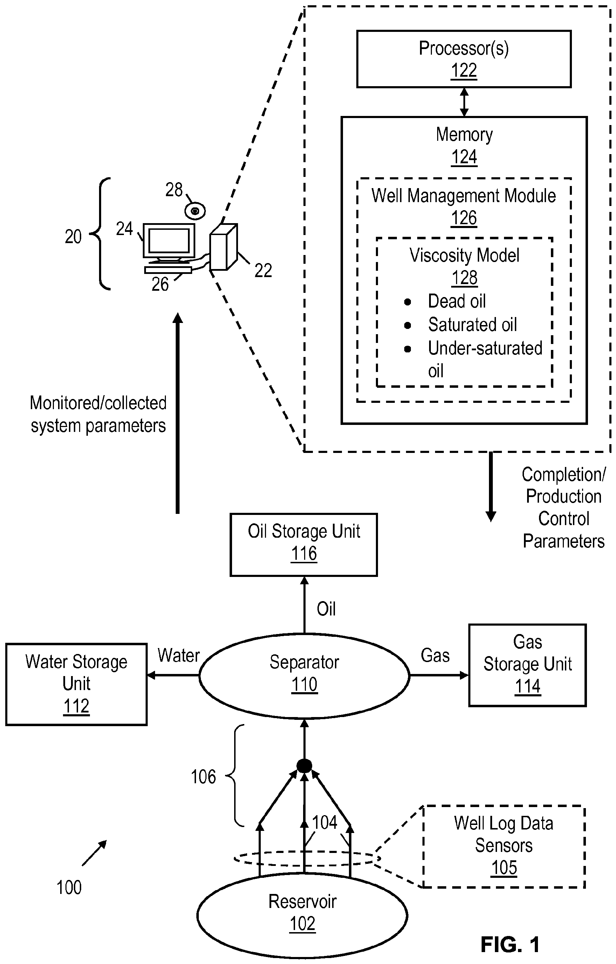

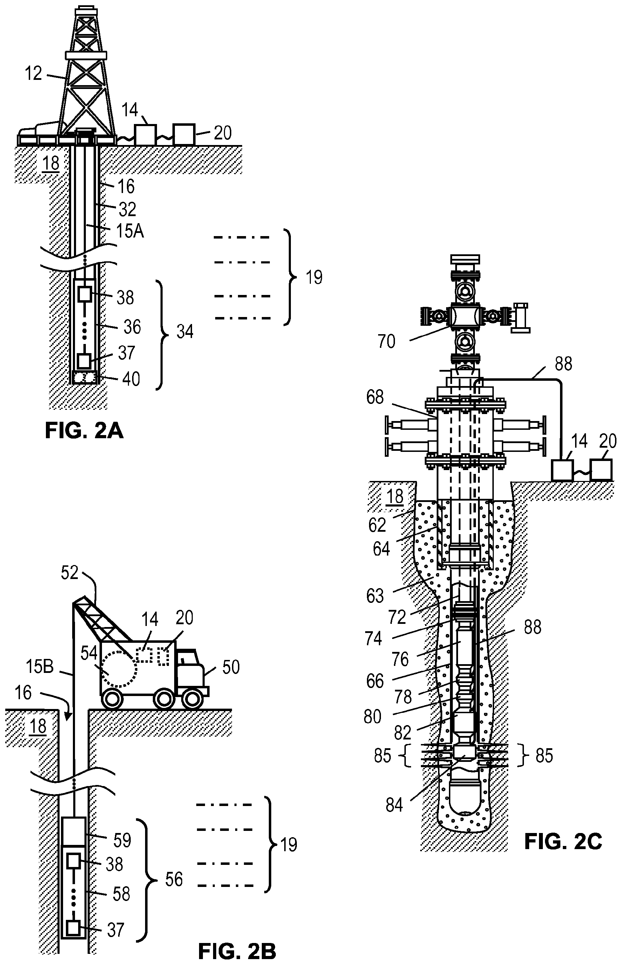

FIG. 1 shows an illustrative hydrocarbon production system 100. The illustrated hydrocarbon production system 100 includes a plurality of wells 104 extending from a reservoir 102, where the arrows representing the wells 104 show the direction of fluid flow (*i.e.,* wells 104 represent production wells). Although only production wells are shown, the hydrocarbon production system 100 could also include injector wells and/or monitoring wells. Further, the hydrocarbon production system 100 also includes well log data sensors 105 associated with one or more of the wells 104. Such well log data sensors may correspond to one or more of the logging survey environments described herein (*see e.g.,* FIGS. 2A-2C).

In FIG. 1, a surface network 106 transports fluid from the wells 104 to a separator 110, which directs water, oil, and gas to separate storage units 112, 114, and 116. The water storage unit 112 may direct collected water back to reservoir 102 or elsewhere. The gas storage unit 114 may direct collected gas back to reservoir 102, to a gas lift interface, or elsewhere. The oil storage unit 116 may direct collected oil to one or more refineries. In different embodiments, the separator 110 and storage units 112, 114, and 116 may be part of a single facility or part of multiple facilities associated with the hydrocarbon production system model 100. Although only one oil storage unit 116 is shown, it should be understood that multiple oil storage units may be used in the hydrocarbon production system 100. Similarly, multiple water storage units 112 and/or multiple gas storage units 114 may be used in the hydrocarbon production system 100.

In FIG. 1, the hydrocarbon production system 100 includes a computer system 20 for performing data analysis and directing operations of the system 100. In at least some embodiments, the computer system 20 includes a processing unit 22 that performs the viscosity estimation operations described herein by executing software or instructions obtained from a local or remote non-transitory computer-readable medium 28. In at least some embodiments, viscosity estimation operations are based at least in part on monitored or collected system parameters. In particular, temperature, pressure, oil-to-gas ratio, and density measurements may be useful for calculating crude oil viscosity. The computer system 20 also may include input device(s) 26 (*e.g.,* a keyboard, mouse, touchpad, etc.) and output device(s) 24 (*e.g.,* a monitor, printer, etc.). Such input device(s) 26 and/or output device(s) 24 provide a user interface that enables an operator to interact with the logging tool 36 and/or software executed by the processing unit 22. For example, the computer system 20 may enable an operator to select data analysis options (including options for calculating crude oil viscosity), to view collected measurements, to view analysis results, and/or to perform other tasks.

In at least some embodiments, the computer system 20 uses a calculated crude oil viscosity to perform fluid flow simulations related to pre-production, production, and/or post-production operations. Further, the computer system 20 may use the calculated crude oil viscosity and/or simulation results to direct well completion operations, production settings (*e.g.,* gas lift), and/or enhanced oil recovery settings.

In FIG. 1, the processing unit 22 is shown to include processor(s) 122 and a memory 124 storing a well design/management module 126 with a viscosity model 128. The memory 124 corresponds to one or more known variations of non-transitory computer-readable media (*e.g.,* optical disc, random access memory (RAM), Flash drives, etc.). When executed, the well design/management module 126 performs various operations including calculating crude oil viscosity using viscosity model 128. Calculated viscosity values may be stored (*e.g.,* in memory 124 or elsewhere) for use with fluid flow simulations and/or to direct well completion operations, production operations (*e.g.,* gas lift), enhanced oil recovery operations, and/or other operations. Such simulations and operations may be performed by well design/management module 126 and/or other software. As examples, well design/management module 126 may correspond to versions of WELLCAT™, DecisionSpace Well Engineering®, WELLPLAN™, or other engineering software.

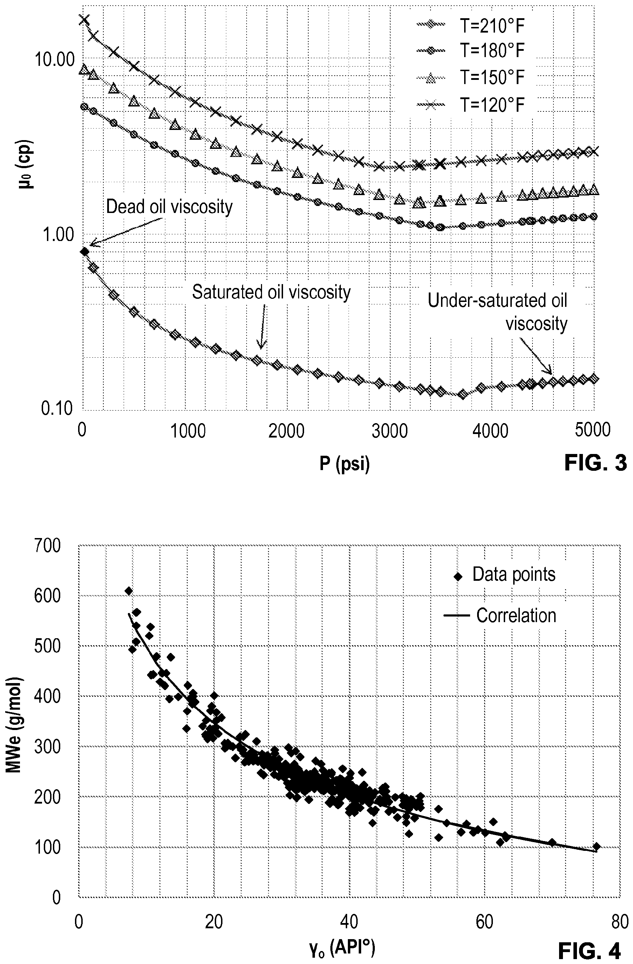

The viscosity model 128 enables calculation of crude oil viscosity for HPHT conditions that are becoming more common (*e.g.,* offshore deep-water wells and unconventional oil wells such as steam-assisted gravity drain wells). In at least some embodiments, the viscosity model 128 covers all kinds of crude oils ranging from gas condensate, volatile oil, black oil, heavy oil to oil-sand bitumen. FIG. 3 shows an illustrative chart of crude oil viscosity changes with temperature and pressure. In FIG. 3, viscosity curves as a function of pressure are shown for four different temperatures. Different portions of each viscosity curve correspond to dead oil viscosity, saturated oil viscosity, and under-saturated oil viscosity.

Without limitation to other techniques, the state of crude oil may be identified as dead oil, saturated oil, or under-saturated oil based on a temperature measurement, a pressure measurement, and an oil/gas ratio measurement. In accordance with at least some embodiments, the viscosity model 128 includes adjustments depending on the state of the crude oil as well as the availability of crude oil parameters (*e.g.*, a Watson characterization factor) and/or log data.

In an example embodiment, the viscosity model 128 is based on a model introduced by Yarranton et al., which relies on gas-chromatography (GC) to predict crude oil viscosity. See Yarranton et al., Cold, Hot, or Dilute: Modeling the Viscosity of Heavy Oil for In Situ Processes, GeoConvention 2013: Integration. In the Yarranton model, viscosity is estimated as:

$$\mu = \mu_0[1 + \alpha(T)\Delta P], \qquad (1)$$

where $\alpha(T)=(0.008+0.00006MW)(1-0.0033\Delta T)$, and where $\mu_o$ is the oil viscosity at atmospheric pressure and temperature T.

In at least some embodiments, $\mu_o$ is calculated using the following equations:

$$log[log(\mu_0 + 1)] = A - Blog(T), \qquad (2)$$

$$A = k_1(1 - exp(-k_2 MW) + k_3 MW, \qquad (3)$$

$$B = k_4(1 - exp(-k_5 MW), \qquad (4)$$

where $MW$ is the molecular weight of crude oil, and $k_1$ to $k_5$ are predetermined constants. As an example, suitable values for $k_1$ to $k_5$ are: $k_1 = 9.77$; $k_2 = 0.01$; $k_3 = 0.00028$; $k_4 = 3.71$; and $k_5 = 0.015$.

In at least some embodiments, the value for MW in equations 1, 3, and 4 corresponds to an effective molecular weight ($MW_{EFF}$) determined by correlating the relative density of crude oil with molecular weight (*i.e.,* GC or other expensive analysis is not needed). One example relative density is referred to as American Petroleum Institute (API) gravity, and according to the present invention, the effective molecular weight used in equations 1 to 4 is determined as:

$$MW_{EFF} = k_6 + k_7\gamma_{API} - (k_8 - k_9\gamma_{API})ln(\gamma_{API}), \qquad (5)$$

where $\gamma_{API}$ is an API gravity value for the crude oil, and $k_6$ to $k_9$ are predetermined constants. As an example, suitable values for $k_6$ to $k_9$ are: $k_6 = 1006.7$; $k_7 = 0.38$; $k_8 = 222.6$; and $k_9 = 0.237$. FIG. 4 shows an illustrative chart correlating effective molecular weight with API gravity. In at least some embodiments, equations 1-5 are applied by the well design/management module 126 to calculate viscosity for crude oil in a dead oil state. The performance of the viscosity model 128 relative to the Beal-Standing model is given in Table 1.

**Table 1**

| Model | Absolute Relative Deviation (%) | |
|---|---|---|
| | Average | Maximum |
| Viscosity Model 128 | 47 | 606 |
| Beal-Standing | 78 | 2386 |

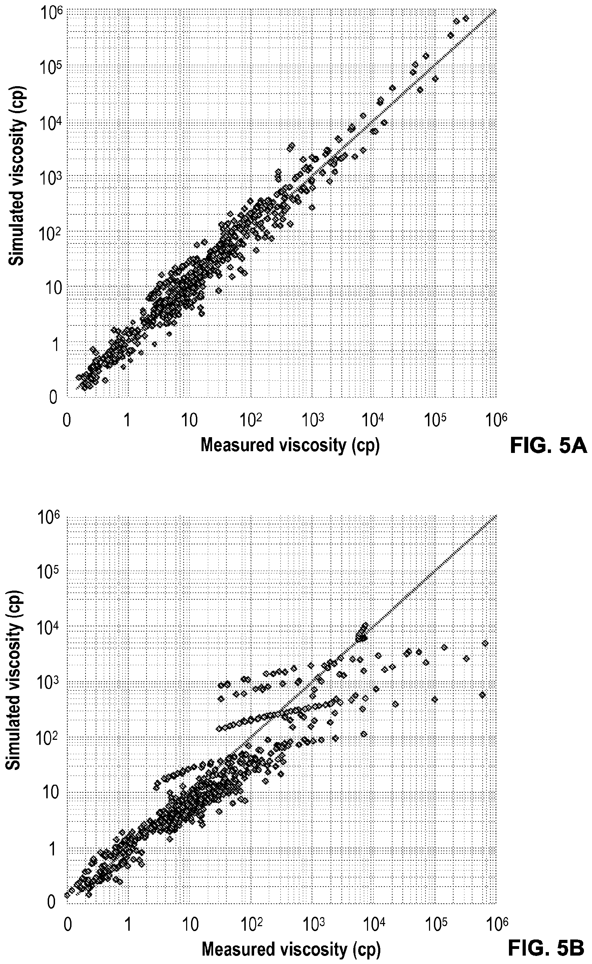

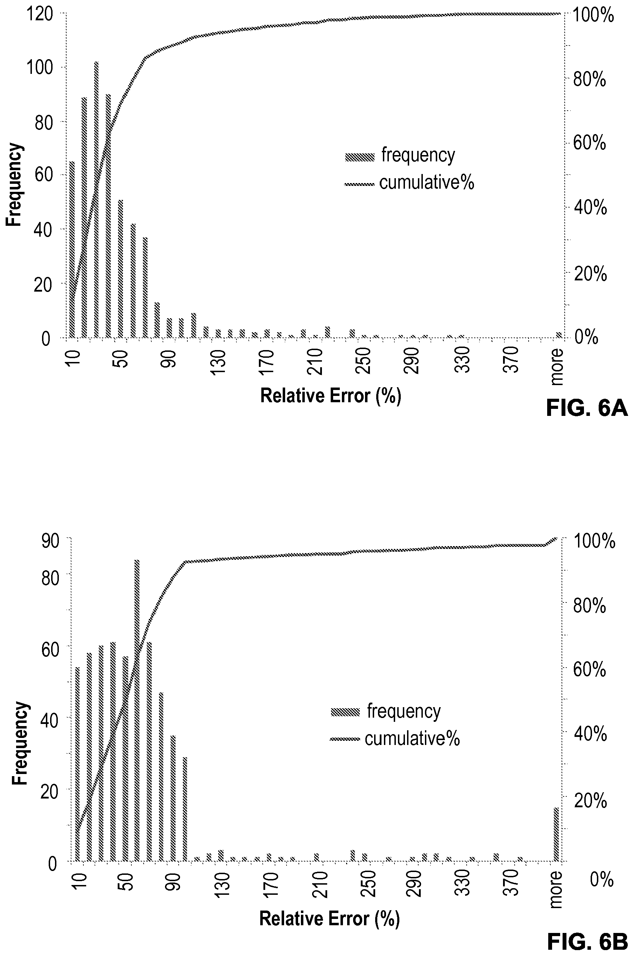

In addition to the performance comparison of Table 1, FIGS. 5A, 5B, 6A, and 6B show charts illustrating the performance of the viscosity model 128 relative to the Beal-Standing model. More specifically, the charts of FIGS. 5A and 6A show prediction accuracy of the viscosity model 128, while the charts of FIGS. 5B and 6B show prediction accuracy of the Beal-Standing model. Compared to the Beal-Standing model, the viscosity model 128 has improved accuracy for higher values of viscosity that are common in heavy oils and/or high-pressure scenarios.

A Watson characterization factor or K-factor is a systematic way of classifying a crude oil according to its paraffinic, naphthenic, intermediate or aromatic nature. 12.5 or higher indicate a crude oil of predominantly paraffinic constituents, while 10 or lower indicate a crude of more aromatic nature. Paraffinic (linear) hydrocarbon molecules are subject to more intermolecular interactions and are consequently more viscous. Incorporation of the Watson characterization factor ($K_w$) into viscosity models has been shown to improve estimation accuracy. See e.g., Bergman et al., A Consistent and Accurate Deal-Oil-Viscosity Method, SPE Reservoir Evaluation & Engineering, vol. 12, issue 6, pages 815-840 (2009). In at least some emboidments, the viscosity model 128 provides an option of using $K_w$, if available. For example, the viscosity model 128 may employ an adjustment of effective MW ($\Delta MW_{EFF}$) that includes the effect of $K_w$ is given as:

$$\Delta MW_{EFF} = k_{10}K_w - k_{11}, \tag{6}$$

where $k_{10}$ and $k_{11}$ are predetermined constants. As an example, suitable values for $k_{10}$ and $k_{11}$ are:

$k_{10} = 584.41 \times \gamma_{API}^{-0.655}$ and $k_{11} = 6545.26 \times \gamma_{API}^{-0.632}$. The performance of the viscosity model 128 with and without the $K_w$ effect is given in Table 2.

Table 2

| Viscosity Model 128 option | Absolute Relative Deviation (%) | |
|---|---|---|
| | Average | Maximum |
| With $K_w$ | 31.16 | 230.9 |
| Without $K_w$ | 114.12 | 1647.22 |

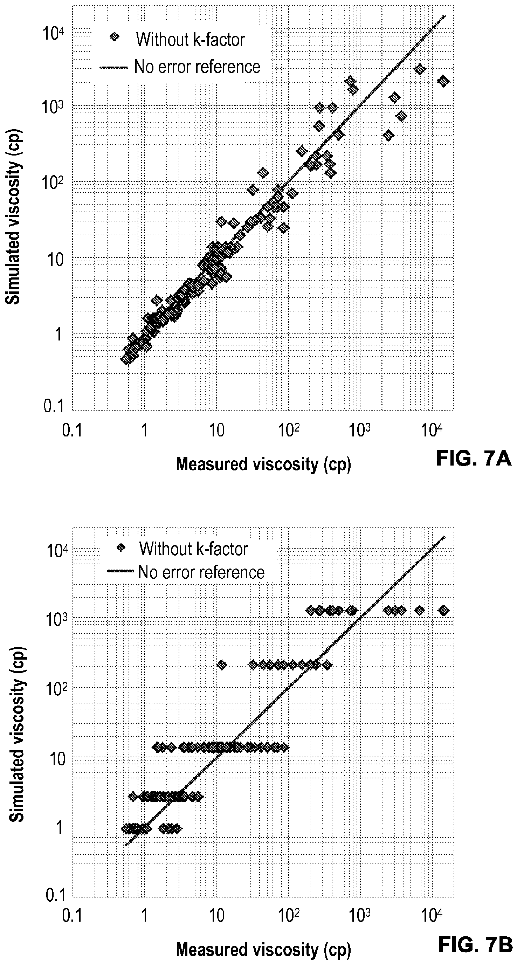

In addition to the performance comparison of Table 2, FIGS. 7A and 7B show charts illustrating the performance of the viscosity model 128 with and without the $K_w$ effect. More specifically, the chart of FIG. 7A shows prediction accuracy of the viscosity model 128 with the $K_w$ effect, while the chart of FIG. 7B shows prediction accuracy of the viscosity model 128 without the $K_w$ effect.

The viscosity model 128 also may account for the effect of solution gas (*i.e.,* a saturated or live oil scenario). This is done by calculating an effective molecular weight with gas content adjustment determined using available oil field data *(e.g.,* gas gravity, gas/oil ratio, oil API gravity). For example, an effective molecular weight with gas content adjustment ($MW_{EFF,GCA}$) may be calculated as:

$$MW_{EFF,GCA} = MW_{EFF} \times x_0 + MW_{gas} \times (1 - x_0) \times n, \tag{7}$$

where $MW_{EFF}$ is calculated from equation 5, $x_0$ is the deal oil molar fraction in the gas saturated oil, $MW_{gas}$ is the gas molecular weight, and $n$ is the non-ideality factor to account for the mixing effect of gas and deal oil. If $x_0$ is expressed in terms of solution gas/oil ratio ($R_s$) and gas gravity ($\gamma_g$), an effective molecular weight with gas content adjustment ($MW_{EFF,GCA}$) may be calculated as:

$$MW_{EFF,GCA} = MW_{EFF} \times \frac{\rho_0 + nR_s\rho_{air}\gamma_g}{\rho_0 + R_s\rho_{air}\frac{MW_{EFF}}{MW_{air}}}, \tag{8}$$

where $MW_{EFF}$ is calculated from equation 5, $\rho_0$ is a density value of the crude oil (*e.g.,* $\rho_0$ = 141.5/(131.5 + *oil API gravity*) $g/cm^3$), $n$ is a non-ideality factor (*e.g.,* obtained from a regression process), $R_s$ is a gas/oil ratio at bubble point pressure, $\rho_{air}$ is a density value of air at standard condition (*e.g.,* 0.001225 $g/cm^3$), $\gamma_g$ is a density value of gas, and $MW_{air}$ is a molecular weight value of air (*e.g.,* 29 $g/mol$).

Accordingly, in at least some embodiments, the viscosity model 128 may estimate viscosity for a saturated crude oil scenario using equation 1-5 and 8 and available crude oil measurements. More specifically, the *MW* value for equations 1, 3, and 4 may correspond to a $MW_{EFF,GCA}$ value determined using equation 8 for a gas/oil ratio at bubble point pressure. In equation 8, the $MW_{EFF}$ value is calculated using equation 5 (correlating a molecular weight with available relative density measurements). Further, the $MW_{EFF}$ value used for equation 8 may be adjusted using equation 6 (a Watson characterization adjustment).

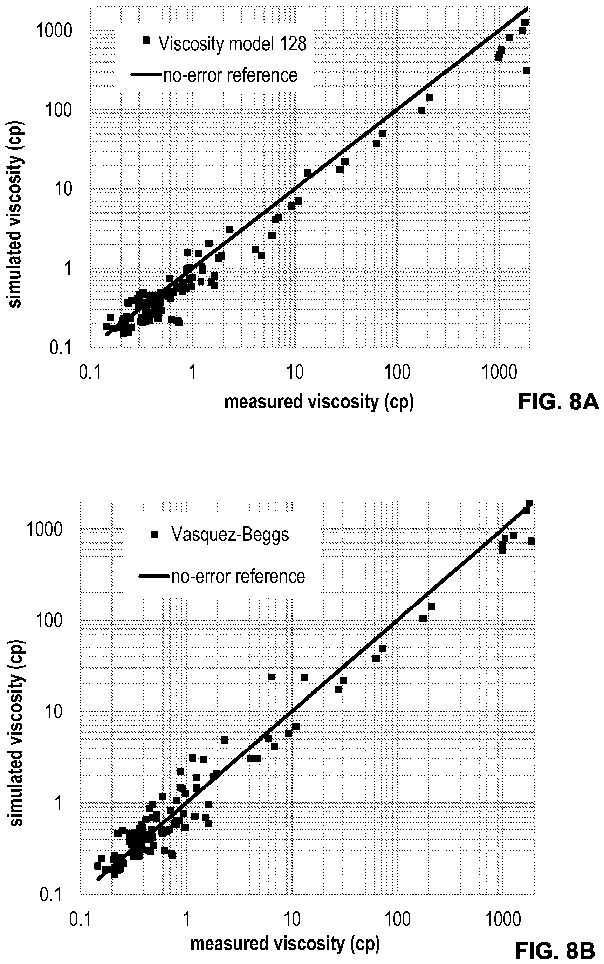

FIG. 8A shows an illustrative chart showing accuracies of viscosity model 128 for live oil (*e.g.*, equation 8) based on bubble point viscosity data from various wells. Meanwhile, FIG. 8B shows an illustrative chart showing accuracies of a Vasquez-Beggs viscosity model for live oil. The average absolute relative error for viscosity model 128 is 29%, and the maximum absolute relative error is 83%. In contrast, the average absolute relative error for the Vasquez-Beggs viscosity model is 35%, and the maximum error is 271%. For the viscosity model 128, the value of $n$ (the non-ideality factor) may be further tuned as a function of API gravity, gas gravity, or other available parameters to improve model accuracy.

The viscosity model 128 also may account for HPHT scenarios, where crude oil is often under-saturated. In at least some embodiments, the viscosity model 128 calculates viscosity for an under-saturated crude oil scenario as:

$$\mu = \mu_{ob}exp[k_{12} \times (P - P_b)/\gamma_{API}], \qquad (9)$$

where $\mu_{ob}$ is a crude oil viscosity value corresponding to an effective molecular weight with gas content adjustment for a gas/oil ratio at bubble point pressure, $k_{12}$ is a predetermined constant, $P$ is a measured pressure of the crude oil, $P_b$ is a bubble point pressure of the crude oil, and $\gamma_{API}$ is an API gravity value for the crude oil. As an example, a suitable value for $k_{12}$ is $k_{12} = 0.0015$ (e.g., determined from a regression process). In at least some embodiments, $\mu_{ob}$ is calculated using equations 1-5 and 8 (i.e., $\mu_{ob}$ is the calculated saturated oil viscosity). Accordingly, equation 9 operates to scale the saturated oil viscosity calculated using equations 1-5 and 8 based on a relative pressure adjustment ($P - P_b$) that identifies how close to bubble point pressure the crude oil is at.

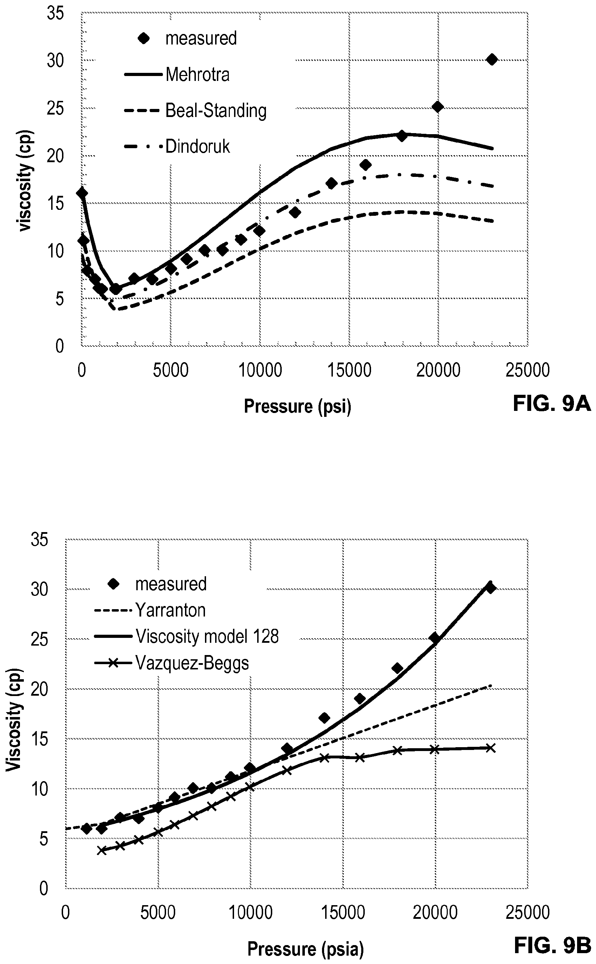

FIG. 9A and 9B show an illustrative chart showing deviation of existing viscosity models at HPHT conditions. More specifically, FIG. 9A shows that viscosity estimates using a Mehrotra model, a Beal-Standing model, and a Dindoruk model deviate from measured viscosity values, particularly at higher pressures. Similarly, FIG. 9B shows that viscosity estimates using a Yarranton model, and Vazquez-Beggs model deviate from measured viscosity values, particularly at higher pressures. In contrast, the viscosity estimates using the viscosity model 128 follow measured viscosity values quite well even at higher pressures (see FIG. 9B).

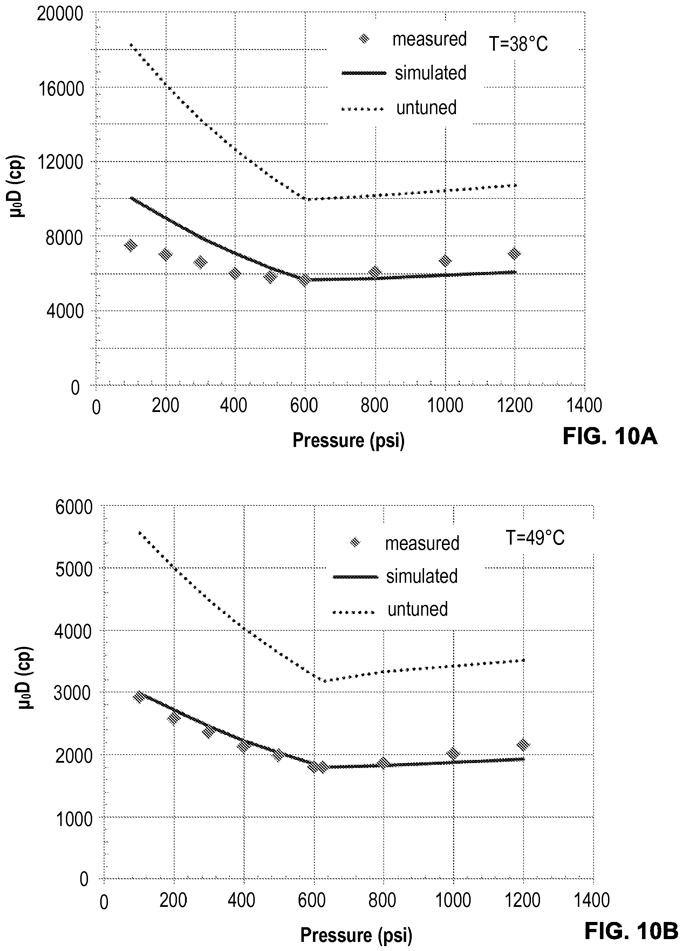

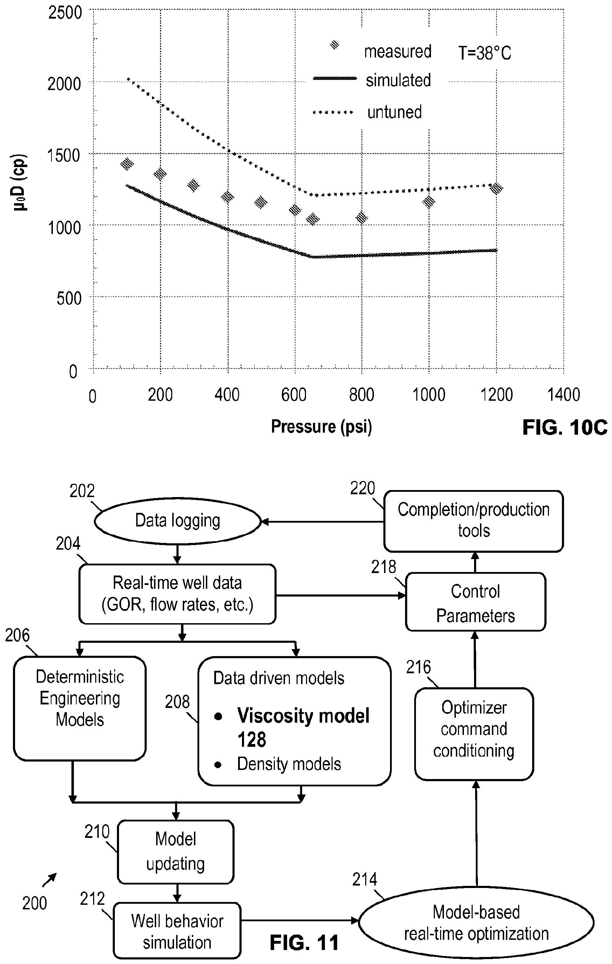

FIGS. 10A-10C show illustrative charts showing predicted viscosity values at different pressures and temperatures. For FIGS. 10A-10C, the effective molecular weight was tuned to match viscosity data at T=38 °C, P=600 psia, and oil API gravity = 8.48. The result of the tuning operation was an effective molecular weight value of 508.24 compared to a calculated molecular weight value of 531. For FIGS. 10A-10C, the single data-point tuning results in an average absolute relative error of 14.38% and a maximum absolute relative error of 34.17%. Without tuning, the average absolute relative error is 65% and the maximum absolute relative error is 143.5%. Accordingly, in at least some embodiments, the results of the viscosity model 128 may be tuned using a single data-point tuning to improve accuracy.

FIG. 11 shows an illustrative workflow 200 for employing the viscosity model 128. In workflow 200, data logging operations are performed at block 202 to obtain real-time well data 204 such as gas/oil ratios, flow rates, gas injection rates, produced oil rate, water cut, produced gas rate, etc. To provide some context for the data logging operations of block 202, FIGS. 2A-2C show illustrative survey environments including a logging-while-drilling (LWD) survey environment, a wireline logging survey environment, and permanent well survey environment.

FIG. 2A shows an illustrative LWD survey environment, in which a drilling assembly 12 enables a wired drill string 32 to be lowered and raised in a borehole 16 that penetrates formations 19 of the earth 18. At the lower end of the wired drill string 32, a bottomhole assembly 34 with a drill bit 40 removes material and penetrates formations 19 using known drilling techniques. The bottomhole assembly 34 also includes a logging tool 36 with sensor(s) 38 to obtain measurements in the downhole environment. Example collected measurements or derived measurements include temperature, pressure, gas/oil ratios and fluid flow rates. For some types of measurements, the logging tool 36 includes transmitter(s) 37 to emit a source signal, where sensor(s) 38 measures how the downhole environment reflects or otherwise modifies the source signal. The logging tool 36 may also include electronics for data storage, communication, etc. The measurements obtained by sensor(s) 38 are conveyed to earth's surface and/or are stored by the logging tool 36. In either case, measurements as a function of position and/or time may be analyzed to determine properties of formations 19 and/or corresponding fluids. For example, the measurements obtained from sensor(s) 38 may be used to derive a log of temperature, pressure, gas/oil ratios and/or fluid flow rates as a function of position or time. The logs and/or formation properties may be displayed to an operator. Further, such logs and/or formation properties may be used by a well design/management module 126 to improve viscosity estimates of the viscosity model 128.

In FIG. 2A, a cable 15A is represented. The cable 15A may take different forms and includes embedded electrical conductors and/or optical waveguides (e.g., fibers) to enable interrogation of sensor(s) 38, as well as transfer of power and/or communications between the bottomhole assembly 34 and earth's surface. The cable 15A may be integrated with, attached to, or inside the drill string 32. In at least some embodiments, cable 15A may be supplemented by or replaced at least in part by mud pulse telemetry or other wireless communication techniques.

In FIG. 2A, an interface 14 at earth's surface receives the measurements via cable 15A or another telemetry channel and conveys the measurements to a computer system 20. In some embodiments, the surface interface 14 and/or the computer system 20 may perform various operations such as converting received signals from one format to another, storing measurements from sensors(s) 38, processing the measurements, deriving logs from the measurements, and/or displaying logs or other measurement data. Further, the computer system 20 (or another computer system) may execute well design/management software (e.g., well design/management module 126) with a viscosity model 128 as described herein. The viscosity estimates calculated using the viscosity model 128 may be displayed to an operator and/or may be used for other operations such as fluid flow simulations, model updates, well completion operations, production operations, etc.

At various times during the drilling process, the drill string 32 shown in FIG. 2A may be removed from the borehole 16. With the drill string 32 removed, wireline logging operations may be performed as shown in the wireline logging survey environment of FIG. 2B. In FIG. 2B, a wireline logging string 56 is suspended in borehole 16 that penetrates formations 19 of the earth 18. For example, the wireline logging string 56 may be suspended by a cable 15B having conductors and/or optical fibers for conveying power to the wireline logging string 56. The cable 15B may also be used as a communication interface for uphole and/or downhole communications. For example, the cable 15B may be used to interrogate the sensor(s) 38 or otherwise enable uphole transmissions of measurements collected by sensor(s) 38. In at least some embodiments, the cable 15B wraps and unwraps as needed around cable reel 54 when lowering or raising the wireline logging string 56. As shown, the cable reel 54 may be part of a movable logging facility or vehicle 50 having a cable guide 52.

The wireline logging string 56 includes logging tool(s) 59 and a logging tool 58 with sensor(s) 38 and optional source(s) 37 to obtain measurements. The logging tool 58 may also include electronics for data storage, communication, etc. The measurements obtained by sensor(s) 38 are conveyed to earth's surface and/or are stored by the logging tool 58. As previously noted, such measurements as a function of position or time may be analyzed to determine formation properties, fluid properties, and/or fluid flow properties as described herein. At earth's surface, a surface interface 14 receives the measurements via the cable 15B or other telemetry, and conveys the measurements to computer system 20, or another computer system, for analysis including calculating viscosity estimates using viscosity model 128.

FIG. 2C shows a well installation survey environment corresponding to a production well, where a borehole 62 has been drilled into the earth 18. Such boreholes 62 may be drilled to ten thousand feet or more in depth and can be steered horizontally for perhaps twice that distance. The production well also includes a casing header 64 and casing 66, both secured into place by cement 63. The casing 66 is typically formed from tubular casing sections (usually about 30-40 feet long) connected end-to-end. Blowout preventer (BOP) 68 couples to the casing header 64 and to production wellhead 70, which together seal in the well head and enable fluids to be extracted from the well in a safe and controlled manner.

Measurement data is periodically sampled and collected from the production well of FIG. 2C and combined with measurements from other wells within a reservoir, enabling the overall state of a reservoir to be monitored and assessed. These measurements may be taken using a number of different downhole and surface instruments, including but not limited to, temperature and pressure sensor 78 and flow meter 80. Additional devices also coupled in-line to production tubing 72 include downhole choke 76 (used to vary the fluid flow restriction), electric submersible pump (ESP) 82 (which draws in fluid flowing from perforations 85 outside ESP 82 and production tubing 72), ESP motor 84 (to drive ESP 82), and packer 74 (isolating the production zone below the packer from the rest of the well). Additional surface measurement devices may be used to measure, for example, the tubing head pressure and the electrical power consumption of ESP motor 84. In other production wells, a gas lift injector mandrel is coupled in-line with production tubing 72 to control injected gas flowing into the production tubing at the surface.

In at least some embodiments, the various sensors of the production well of FIG. 2C couple to a cable 88 that runs along production tubing 72. For example, the cable 88 may be attached to the exterior of production tubing 72 (e.g., by bands) and exits through blowout preventer 68, where it couples to surface interface 14. Cable 88 may provide power to downhole components to manage production operations and/or enhanced oil recovery operations. Further, the cable 88 provides signal paths (electrical, optical, etc.) to enable uphole or downhole communications between downhole components/sensors of the production well and surface computer 20. The production well of FIG. 2C may be controlled and monitored locally by field personnel using a user interface built into local computer system (e.g., computer system 20), or may be controlled and monitored by a remote computer system in communication with the computer system 20 or surface interface 14. Communications between the computer system 20 or surface interface 14 and a remote computer system may be wired and/or wireless.

In at least some embodiments, additional measurement data may be collected using a production logging tool, which may be lowered by a cable into production tubing 72. In other illustrative embodiments, production tubing 72 is first removed, and the production logging tool is then lowered into casing 66. In other alternative embodiments, coil tubing may be used to lower and raise a production logging tool. Such production logging tools may be pushed down either the production tubing 72 or the casing 66 with the production tubing 72 removed. The additional measurement data obtained from such production logging tools can be used to supplement other downhole sensors. The additional measurement data may be communicated to computer system 20, or another computer system, during the logging process, or alternatively may be retrieved after the tool assembly is removed from the downhole environment.

Returning to FIG. 11, the real-time well data 204 obtained from the data logging operations of block 202 (*e.g.,* from one or more of the survey environments of FIGS. 2A-2C) is provided to deterministic engineering model 206 as well as data driven models 208, include the viscosity model 128. As an example, the real-time well data 204 may be used to update the effective molecular weight used by the viscosity model 128 to estimate viscosity. The viscosity model 128 and the other data driven models 208 and/or deterministic engineering models 206 are used to perform model updating at block 210. For example, a well model and/or a reservoir model may be updated at block 210. With the model updates from block 210, well behavior simulations 212 (*e.g.,* production rates, production cost, well integrity, etc.) are performed. At block 214, model-based real-time optimization operations are performed. At block 214, optimization criteria (*e.g.,* maximum gross profit) are applied to determine operating parameters such as gas injection rates, surface injection rates, etc. With the optimizations of block 214, an optimizer command conditioning block 216 determines control parameters 218 for available well completion and/or production tools 220. The completion/production tools 220 apply the control parameters 218 to perform operations to update fluid flow for a particular well, for multiple wells, or for an entire reservoir. To summarize, the viscosity model 128 described herein may be part of a workflow (*e.g.,* workflow 200) that optimizes control parameters for wells, a surface network, a separator, a water storage unit, a gas storage unit, an oil storage unit, gas lift units, or other components of a hydrocarbon production system (e.g., system 100). The optimization of such control parameters may involve comparing measured data with simulated data, where the simulated data is based at least in part on viscosity values estimated using the viscosity model 128. With the improved accuracy of viscosity model 128, the amount/frequency of such simulations and comparisons can be reduced compared to using other viscosity models.

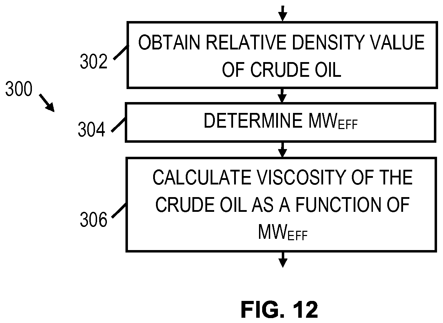

FIG. 12 shows an illustrative method 300 for estimating viscosity. The method 300, may be performed, for example, by a computer system (*e.g.,* computer system 20) that executes software including viscosity model 128 described herein. In method 300, a relative density value of crude oil is obtained at block 302. For example, the relative density value may be obtained from log data collected in one or more wells. In the present invention, the relative density value corresponds to API gravity. At block 304, an effective molecular weight is determined. For example, in at least some embodiments, equation 5 or a similar correlation curve is used to correlate an effective molecular weight with relative density. At block 306, viscosity of the crude oil is calculated as a function of the effective molecular weight (*see e.g.,* equations 1-5). As described herein, the viscosity model 128 allows for adjustments. For example, the viscosity calculation of block 306 may be adjusted by adjusting the effective molecular weight using a Watson characterization adjustment, a gas content adjustment, a relative pressure adjustment, a single data-point tuning adjustment, and/or other adjustments (*see e.g.,* equations 6-9 and FIGS. 10A-10C).

The viscosity model 128 described herein can be understood to be a universal model. In other words, viscosity model 128 is both a compositional model and an extended black oil model. With the viscosity model 128, oil composition data and/or oil field property data (*e.g.,* API gravity, gas/oil ratio, etc.) can be used to calculate oil viscosity. Unlike many other models that were developed using oil data for a particular geological region (*e.g.,* Middle East or Gulf of Mexico), the viscosity model 128 was developed using data from worldwide oils. Accordingly, implementation and maintenance of the viscosity model 128 should be simplified compared to model developed using a narrower set of oil data. The viscosity model 128 is the first known attempt to use effective molecular weight tuned by solution gas/oil ratio and gas gravity data to model saturated oil viscosity. This tuning provides greater simplicity and potentially higher accuracy compared to other models. Further, the viscosity model 128 is the first known attempt to extend a black oil viscosity model into HPHT conditions. The accuracy of viscosity model 128 can be significantly improved by tuning with only one measured viscosity value (*see e.g.,* FIGS 10A-10C). Other models usually require multiple data points for calibration. The viscosity model 128 covers a very broad oil API gravity range from 7.3 to 75 °API. Although not validated with real data, the viscosity model 128 still yields physically meaningful results when the API gravity range is extended to [1.0, 145]. Other viscosity models usually cover a smaller API gravity range (*e.g.,* from 15 to 50 °API) and may generate physically impossible results. With the viscosity model 128, the accuracy, robustness, and speed of well behavior simulations (*e.g.,* to predict pressure and temperature) are improved. Various software applications (*e.g.,* WELLCAT™, NETool™, Nexus®, etc.) may employ the viscosity model 128 for well behavior simulations or other operations.

Embodiments disclosed herein include:

A: A method that comprises obtaining a relative density value of crude oil, and determining an effective molecular weight of the crude oil based on the relative density value. The method also comprises calculating a crude oil viscosity value as a function of the effective molecular weight. The method also comprises storing or displaying the crude oil viscosity value.

B. A system that comprises a memory unit that stores a relative density value of crude oil, and at least one processing unit that determines an effective molecular weight of crude oil based on the relative density value. The at least one processing unit calculates a crude oil viscosity value as a function of the effective molecular weight.

Each of the embodiments, A and B, may have one or more of the following additional elements in any combination. Element 1: the effective molecular weight ($MW_{EFF}$) is determined using: $MW_{EFF} = k_6 + k_7\gamma_{API} - (k_8 - k_9\gamma_{API})ln(\gamma_{API})$, where $\gamma_{API}$ is an API gravity value for the crude oil, and $k_6$ to $k_9$ are predetermined constants. Element 2: determining the effective molecular weight comprises applying a Watson characterization adjustment. Element 3: the Watson characterization adjustment is represented as: $\Delta MW_{EFF} = k_{10}K_w - k_{11}$, where $\Delta MW_{EFF}$ is a change in the effective molecular weight, $K_w$ is a Watson characterization factor, and $k_{10}$ and $k_{11}$ are predetermined constants. Element 4: determining the effective molecular weight comprises applying a gas content adjustment. Element 5: the effective molecular weight with gas content adjustment ($MW_{EFF,GCA}$) is represented as:

$$MW_{EFF,GCA} = MW_{EFF} \times \frac{\rho_0 + nR_s\rho_{air}\gamma_g}{\rho_0 + R_s\rho_{air}\frac{MW_{EFF}}{MW_{air}}},$$

where $MW_{EFF}$ is an effective molecular weight value based on the obtained relative density of the crude oil, $\rho_0$ is a density value of the crude oil, $n$ is a non-ideality factor, $R_s$ is a gas/oil ratio at bubble point pressure, $\rho_{air}$ is a density value of air, $\gamma_g$ is a density value of gas, and $MW_{air}$ is a molecular weight value of air. Element 6: determining the effective molecular weight comprises applying a gas content adjustment and a relative pressure adjustment. Element 7: the relative pressure adjustment is based on a comparison of a measured pressure of the crude oil with a bubble point pressure of the crude oil. Element 8: the crude oil viscosity value ($\mu$) is calculated as: $= \mu_{ob}exp[k_{12} \times (P - P_b)/\gamma_{API}]$, where $\mu_{ob}$ is a crude oil viscosity value corresponding to an effective molecular weight with gas content adjustment for a gas/oil ratio at bubble point pressure, $k_{12}$ is a predetermined constant, $P$ is a measured pressure of the crude oil, $P_b$ is a bubble point pressure of the crude oil, and $\gamma_{API}$ is an API gravity value for the crude oil. Element 9: obtaining the relative density value comprises collecting measurements from sensors in a borehole, and deriving an API gravity value for the crude oil based on the collected measurements. Element 10: determining if the crude oil is saturated and applying a gas content adjustment to the effective molecular weight of the crude oil in response to a determination that the crude oil is saturated; and determining if the crude oil is under-saturated and applying a gas content adjustment and a relative pressure adjustment to the effective molecular weight of the crude oil in response to a determination that the crude oil is under-saturated. Element 11: the crude oil viscosity value is calculated by scaling an initial viscosity value ($\mu_0$) represented as: $log[log(\mu_0 + 1)] = A - Blog(T)$, where $A = k_1(1 - exp(-k_2MW) + k_3MW$ and $B = k_4(1 - exp(-k_5MW)$, and where $MW$ is a molecular weight of crude oil, and $k_1$ to $k_5$ are predetermined constants.

Element 12: the at least one processing unit determines the effective molecular weight using: $MW_{EFF} = k_6 + k_7\gamma_{API} - (k_8 - k_9\gamma_{API})ln(\gamma_{API})$, where $MW_{EFF}$ is the effective molecular weight, $\gamma_{API}$ is an API gravity value for the crude oil, and $k_6$ to $k_9$ are predetermined constants. Element 13: the at least one processing unit determines the effective molecular weight by applying a Watson characterization adjustment. Element 14: the at least one processing unit determines the effective molecular weight by applying at least one of a gas content adjustment and a relative pressure adjustment. Element 15: the at least one processing unit calculates the crude oil viscosity value by scaling an initial crude oil viscosity value corresponding to an effective molecular weight with gas content adjustment for a gas/oil ratio at bubble point pressure. Element 16: the at least one processing unit calculates the crude oil viscosity value by scaling a bubble-point crude oil viscosity value based on the effective molecular weight, temperature, and pressure. Element 17: further comprising sensors in a borehole to collect crude oil measurements, wherein the crude oil measurements are used to derive the relative density value of the crude oil. Element 18: the at least one processing unit performs a simulation of fluid movement based at least in part on the calculated crude oil viscosity value. Element 19: the at least one processing unit directs operations of a well completion unit or production unit based at least in part on the calculated crude oil viscosity value.

Numerous variations and modifications will become apparent to those skilled in the art once the above disclosure is fully appreciated. The ensuing claims are intended to cover such variations where applicable.

**Claims**

1.  A computer-implemented method, comprising:

    obtaining an API gravity value as a relative density value of crude oil;
    determining an effective molecular weight of the crude oil based on the relative density value, wherein the effective molecular weight (MW$_{EFF}$) is determined using:

$$MW_{EFF} = k_6 + k_7\gamma_{API} - (k_8 - k_9\gamma_{API})ln(\gamma_{API}),$$

where $\gamma_{API}$ is the obtained API gravity value for the crude oil, and $k_6$ to $k_9$ are predetermined constants; calculating a crude oil viscosity value as a function of the effective molecular weight; and storing or displaying the crude oil viscosity value.

2. The computer-implemented method of claim 1, wherein determining the effective molecular weight comprises applying a Watson characterization adjustment, optionally wherein the Watson characterization adjustment is represented as:

$$\Delta MW_{EFF} = k_{10}K_w - k_{11},$$

where $\Delta MW_{EFF}$ is a change in the effective molecular weight, $K_w$ is a Watson characterization factor, and $k_{10}$ and $k_{11}$ are predetermined constants.

3. The computer-implemented method according to any one of claims 1 or 2, wherein determining the effective molecular weight comprises applying a gas content adjustment, wherein the effective molecular weight with gas content adjustment ($MW_{EFF,GCA}$) is represented as:

$$MW_{EFF,GCA} = MW_{EFF} \times \frac{\rho_0 + nR_s\rho_{air}\gamma_g}{\rho_0 + R_s\rho_{air}\frac{MW_{EFF}}{MW_{air}}},$$

where $MW_{EFF}$ is an effective molecular weight value based on the obtained relative density of the crude oil, $\rho_0$ is a density value of the crude oil, $n$ is a non-ideality factor, $R_s$ is a gas/oil ratio at bubble point pressure, $\rho_{air}$ is a density value of air, $\gamma_g$ is a density value of gas, and $MW_{air}$ is a molecular weight value of air.

4. The computer-implemented method according to any one of claims 1 or 2, wherein determining the effective molecular weight comprises applying a gas content adjustment and a relative pressure adjustment, wherein the relative pressure adjustment is based on a comparison of a measured pressure of the crude oil with a bubble point pressure of the crude oil, and wherein the crude oil viscosity value ($\mu$) is calculated as:

$$\mu = \mu_{ob}exp[k_{12} \times (P - P_b)/\gamma_{API}],$$

where $\mu_{ob}$ is a crude oil viscosity value corresponding to an effective molecular weight with gas content adjustment for a gas/oil ratio at bubble point pressure, which is calculated using the following equations:

$$log[log(\mu_0 + 1)] = A - Blog(T),$$

$$A = k_1(1 - exp(-k_2MW) + k_3MW,$$

$$B = k_4(1 - exp(-k_5MW),$$

wherein MW is the effective molecular weight with gas content adjustment ($MW_{EFF,GCA}$), $k_1$-$k_4$ and $k_{12}$ a predetermined constant, $P$ is a measured pressure of the crude oil, $P_b$ is a bubble point pressure of the crude oil, and $\gamma_{API}$ is an API gravity value for the crude oil.

5. The computer-implemented method according to any one of claims 1 or 2, wherein obtaining the relative density value comprises collecting measurements from sensors in a borehole, and deriving an API gravity value for the crude oil based on the collected measurements.

6. The computer-implemented method according to any one of claims 1 or 2, further comprising:

determining if the crude oil is saturated, based on temperature, pressure and oil/gas ratio measurements, and applying a gas content adjustment to the effective molecular weight of the crude oil in response to a determination that the crude oil is saturated, wherein the effective molecular weight with gas content adjustment ($MW_{EFF,GCA}$) is represented as:

$$MW_{EFF,GCA} = MW_{EFF} \times \frac{\rho_0 + nR_s\rho_{air}\gamma_g}{\rho_0 + R_s\rho_{air}\frac{MW_{EFF}}{MW_{air}}} \;,$$

where $MW_{EFF}$ is an effective molecular weight value based on the obtained relative density of the crude oil, $\rho_0$ is a density value of the crude oil, $n$ is a non-ideality factor, $R_s$ is a gas/oil ratio at bubble point pressure, $\rho_{air}$ is a density value of air, $\gamma_g$ is a density value of gas, and $MW_{air}$ is a molecular weight value of air; and determining if the crude oil is under-saturated, based on temperature, pressure and oil/gas ratio measurements, and applying a gas content adjustment and a relative pressure adjustment to the effective molecular weight of the crude oil in response to a determination that the crude oil is under-saturated, wherein the relative pressure adjustment is based on a comparison of a measured pressure of the crude oil with a bubble point pressure of the crude oil, and wherein the crude oil viscosity value ($\mu$) is calculated as:

$$\mu = \mu_{ob}exp[k_{12} \times (P - P_b)/\gamma_{API}] \,,$$

where $\mu_{ob}$ is a crude oil viscosity value corresponding to an effective molecular weight with gas content adjustment for a gas/oil ratio at bubble point pressure, which is calculated using the following equations:

$$log[log(\mu_0 + 1)] = A - Blog(T),$$

$$A = k_1(1 - exp(-k_2MW) + k_3MW,$$

$$B = k_4(1 - exp(-k_5MW),$$

wherein MW is the effective molecular weight with gas content adjustment ($MW_{EFF,GCA}$), $k_{12}$ is a predetermined constant, $P$ is a measured pressure of the crude oil, $P_b$ is a bubble point pressure of the crude oil, and $\gamma_{API}$ is an API gravity value for the crude oil.

7. The computer-implemented method according to any one of claims 1 or 2, wherein the crude oil viscosity value is calculated by scaling an initial viscosity value ($\mu_0$) represented as:

$$log[log(\mu_0 + 1)] = A - Blog(T),$$

where A = $k_1$(1 - exp(-$k_2$MW) + $k_3$MW and B = $k_4$(1 - exp(-$k_5$MW), and where MW is a molecular weight of crude oil, and $k_1$ to $k_5$ are predetermined constants.

8. A system, comprising:

a memory unit that stores an API gravity value as a relative density value of crude oil; and
at least one processing unit that determines an effective molecular weight of crude oil based on the relative density value, and calculates a crude oil viscosity value as a function of the effective molecular weight, wherein the effective molecular weight ($MW_{EFF}$) is determined using:

$$MW_{EFF} = k_6 + k_7\gamma_{API} - (k_8 - k_9\gamma_{API})ln(\gamma_{API}),$$

where $MW_{EFF}$ is the effective molecular weight, $\gamma_{API}$ is the stored API gravity value for the crude oil, and $k_6$ to $k_9$ are predetermined constants.

9. The system of claim 8, wherein the at least one processing unit determines the effective molecular weight by applying a Watson characterization adjustment.

10. The system according to claim 8, wherein the at least one processing unit determines the effective molecular weight by applying at least one of a gas content adjustment and a relative pressure adjustment.

11. The system of claim 10, wherein the at least one processing unit calculates the crude oil viscosity value by one of:

scaling an initial crude oil viscosity value corresponding to an effective molecular weight with gas content adjustment for a gas/oil ratio at bubble point pressure; and
scaling a standard crude oil viscosity value based on the effective molecular weight, crude oil temperature, and crude oil pressure.

12. The system according to any one of claims 8 to 11, further comprising sensors in a borehole to collect crude oil measurements, wherein the crude oil measurements are used to derive the relative density value of the crude oil.

13. The system according to any one of claims 8 to 11, wherein the at least one processing unit performs a simulation of fluid movement based at least in part on the calculated crude oil viscosity value; or wherein the at least one processing unit directs operations of a well completion unit or production unit based at least in part on the calculated crude oil viscosity value.

**Patentansprüche**

1. Computerimplementiertes Verfahren, umfassend:

Erhalten eines API-Schwerkraftwertes als einen Wert der relativen Dichte von Rohöl;
Bestimmen eines effektiven Molekulargewichts des Rohöls basierend auf dem Wert der relativen Dichte, wobei das effektive Molekulargewicht ($MW_{EFF}$) unter Verwendung des Folgenden bestimmt wird:

$$MW_{EFF} = k_6 + k_7\gamma_{API} - (k_8 - k_9\gamma_{API})ln(\gamma_{API}),$$

wobei $\gamma_{API}$ der erhaltene API-Schwerkraftwert für das Rohöl ist und $k_6$ bis $k_9$ zuvor festgelegte Konstanten sind;
Berechnen eines Rohölviskositätswertes in Abhängigkeit des effektiven Molekulargewichts; und
Speichern oder Anzeigen des Rohölviskositätswertes.

2. Computerimplementiertes Verfahren nach Anspruch 1, wobei das Bestimmen des effektiven Molekulargewichts das Anwenden einer Watson-Charakterisierungsanpassung umfasst, wobei die Watson-Charakterisierungsanpassung optional wie folgt dargestellt ist:

$$\Delta MW_{EFF} = k_{10}K_w - k_{11},$$

wobei $\Delta MW_{EFF}$ eine Änderung des effektiven Molekulargewichts ist, $K_W$ ein Watson-Charakterisierungsfaktor ist und $k_{10}$ und $k_{11}$ zuvor festgelegte Konstanten sind.

3. Computerimplementiertes Verfahren nach einem der Ansprüche 1 oder 2, wobei das Bestimmen des effektiven Molekulargewichts das Anwenden einer Gasgehaltsanpassung umfasst, wobei das effektive Molekulargewicht mit Gasgehaltsanpassung ($MW_{EFF,GCA}$) wie folgt dargestellt ist:

$$MW_{EFF,GCA} = MW_{EFF} \times \frac{\rho_0 + nR_s\rho_{air}\gamma_g}{\rho_0 + R_s\rho_{air}\frac{MW_{EFF}}{MW_{air}}},$$

wobei $MW_{EFF}$ ein Wert des effektiven Molekulargewichts basierend auf der erhaltenen relativen Dichte des Rohöls ist, $\rho_0$ ein Dichtewert des Rohöls ist, $n$ ein Nichtidealitätsfaktor ist, $R_S$ ein Gas-/Öl-Verhältnis bei Siedepunktdruck ist, $\rho_{air}$ ein Dichtewert von Luft ist, $\gamma_g$ ein Dichtewert von Gas ist und $MW_{air}$ ein Molekulargewichtswert von Luft ist.

4. Computerimplementiertes Verfahren nach einem der Ansprüche 1 oder 2, wobei das Bestimmen des effektiven Molekulargewichts das Anwenden einer Gasgehaltsanpassung und einer Anpassung des relativen Drucks umfasst, wobei die Anpassung des relativen Drucks auf einem Vergleich eines gemessenen Drucks des Rohöls mit einem Siedepunktdruck des Rohöls basiert und wobei der Rohölviskositätswert ($\mu$) wie folgt berechnet wird:

$$\mu = \mu_{ob}\,exp[k_{12} \times (P - P_b)/\gamma_{API}],$$

wobei $\mu_{ob}$ ein Rohölviskositätswert entsprechend einem effektiven Molekulargewicht mit Gasgehaltsanpassung für ein Gas-/Öl-Verhältnis bei Siedepunktdruck ist, der unter Verwendung der folgenden Gleichungen berechnet wird:

$$log[log(\mu_0 + 1)] = A - Blog(T),$$

$$A = k_1(1 - exp(-k_2MW) + k_3MW,$$

$$B = k_4(1 - exp(-k_5MW),$$

wobei MW das effektive Molekulargewicht mit Gasgehaltsanpassung ($MW_{EFF,GCA}$) ist, $k_1$-$k_4$ und $k_{12}$ zuvor festgelegte Konstanten sind, P ein gemessener Druck des Rohöls ist, $P_b$ ein Siedepunktdruck des Rohöls ist und $\gamma_{API}$ ein API-Schwerkraftwert für das Rohöl ist.

5. Computerimplementiertes Verfahren nach einem der Ansprüche 1 oder 2, wobei das Erhalten des Wertes der relativen Dichte das Sammeln von Messungen von Sensoren in einem Bohrloch und das Ableiten eines API-Schwerkraftwertes für das Rohöl basierend auf den gesammelten Messungen umfasst.

6. Computerimplementiertes Verfahren nach einem der Ansprüche 1 oder 2, ferner umfassend:

Bestimmen, ob das Rohöl gesättigt ist, basierend auf Messungen der Temperatur, des Drucks und des Öl-/Gas-Verhältnisses, und Anwenden einer Gasgehaltsanpassung auf das effektive Molekulargewicht des Rohöls als Reaktion auf eine Bestimmung, dass das Rohöl gesättigt ist, wobei das effektive Molekulargewicht mit Gasgehaltsanpassung ($MW_{EFF, GCA}$) wie folgt dargestellt ist:

$$MW_{EFF,GCA} = MW_{EFF} \times \frac{\rho_0 + nR_s\rho_{air}\gamma_g}{\rho_0 + R_s\rho_{air}\frac{MW_{EFF}}{MW_{air}}},$$

wobei $MW_{EFF}$ ein Wert des effektiven Molekulargewichts basierend auf der erhaltenen relativen Dichte des Rohöls ist, $\rho_0$ ein Dichtewert des Rohöls ist, $n$ ein Nichtidealitätsfaktor ist, $R_S$ ein Gas-/Öl-Verhältnis bei Siedepunktdruck ist, $\rho_{air}$ ein Dichtewert von Luft ist, $\gamma_g$ ein Dichtewert von Gas ist und $MW_{air}$ ein Molekulargewichtswert von Luft ist; und

Bestimmen, ob das Rohöl untersättigt ist, basierend auf Messungen der Temperatur, des Drucks und des Öl-/Gas-Verhältnisses, und Anwenden einer Gasgehaltsanpassung und einer Anpassung des relativen Drucks auf das effektive Molekulargewicht des Rohöls als Reaktion auf eine Bestimmung, dass das Rohöl untersättigt ist, wobei die Anpassung des relativen Drucks auf einem Vergleich eines gemessenen Drucks des Rohöls mit einem Siedepunktdruck des Rohöls basiert und wobei der Rohölviskositätswert ($\mu$) wie folgt berechnet wird:

$$\mu = \mu_{ob}\, exp[k_{12} \times (P - P_b)/\gamma_{API}]\,,$$

wobei $\mu_{ob}$ ein Rohölviskositätswert entsprechend einem effektiven Molekulargewicht mit Gasgehaltsanpassung für ein Gas-/Öl-Verhältnis bei Siedepunktdruck ist, der unter Verwendung der folgenden Gleichungen berechnet wird:

$$log[log(\mu_0 + 1)] = A - Blog(T),$$

$$A = k_1(1 - exp(-k_2 MW) + k_3 MW,$$

$$B = k_4(1 - exp(-k_5 MW),$$

wobei MW das effektive Molekulargewicht mit Gasgehaltsanpassung ($MW_{EFF,GCA}$) ist, $k_{12}$ eine zuvor festgelegte Konstante ist, $P$ ein gemessener Druck des Rohöls ist, $P_b$ ein Siedepunktdruck des Rohöls ist und $\gamma_{API}$ ein API-Schwerkraftwert für das Rohöl ist.

7.  Computerimplementiertes Verfahren nach einem der Ansprüche 1 oder 2, wobei der Rohölviskositätswert durch Skalieren eines anfänglichen Viskositätswertes ($\mu_0$) berechnet wird, der wie folgt dargestellt ist:

$$log[log(\mu_0 + 1)] = A - Blog(T),$$

wobei A = $k_1$(1 - exp (-$k_2$MW) + $k_3$MW und B = $k_4$(1 - exp(-$k_5$MW) und wobei $MW$ ein Molekulargewicht von Rohöl ist und $k_1$ bis $k_5$ zuvor festgelegte Konstanten sind.

8.  System, umfassend:

    eine Speichereinheit, die einen API-Schwerkraftwert als einen Wert der relativen Dichte von Rohöl speichert; und zumindest eine Verarbeitungseinheit, die ein effektives Molekulargewicht von Rohöl basierend auf dem Wert der relativen Dichte bestimmt und einen Rohölviskositätswert in Abhängigkeit des effektiven Molekulargewichts berechnet, wobei das effektive Molekulargewicht ($MW_{EFF}$) unter Verwendung des Folgenden bestimmt wird:

$$MW_{EFF} = k_6 + k_7\gamma_{API} - (k_8 - k_9\gamma_{API})ln(\gamma_{API}),$$

    wobei $MW_{EFF}$ das effektive Molekulargewicht ist, $\gamma_{API}$ der gespeicherte API-Schwerkraftwert für das Rohöl ist und $k_6$ bis $k_9$ zuvor festgelegte Konstanten sind.

9.  System nach Anspruch 8, wobei die zumindest eine Verarbeitungseinheit das effektive Molekulargewicht bestimmt, indem eine Watson-Charakterisierungsanpassung angewandt wird.

10.  System nach Anspruch 8, wobei die zumindest eine Verarbeitungseinheit das effektive Molekulargewicht bestimmt, indem zumindest eines von einer Gasgehaltsanpassung und einer Anpassung des relativen Drucks angewandt wird.

11.  System nach Anspruch 10, wobei die zumindest eine Verarbeitungseinheit den Rohölviskositätswert durch eines des Folgenden berechnet:

    Skalieren eines anfänglichen Rohölviskositätswertes entsprechend einem effektiven Molekulargewicht mit Gasgehaltsanpassung für ein Gas-/Öl-Verhältnis bei Siedepunktdruck; und
    Skalieren eines standardmäßigen Rohölviskositätswertes basierend auf dem effektiven Molekulargewicht, Rohöltemperatur und Rohöldruck.

**12.** System nach einem der Ansprüche 8 bis 11, ferner umfassend Sensoren in einem Bohrloch, um Rohölmessungen zu sammeln, wobei die Rohölmessungen verwendet werden, um den Wert der relativen Dichte des Rohöls abzuleiten.

**13.** System nach einem der Ansprüche 8 bis 11, wobei die zumindest eine Verarbeitungseinheit eine Simulation von Fluidbewegung basierend zumindest teilweise auf dem berechneten Rohölviskositätswert durchführt; oder wobei die zumindest eine Verarbeitungseinheit Vorgänge einer Bohrlochkomplettierungseinheit oder -produktionseinheit basierend zumindest teilweise auf dem berechneten Rohölviskositätswert anleitet.

**Revendications**

**1.** Procédé mis en œuvre par ordinateur comprenant :

l'obtention d'une valeur de gravité API en tant que valeur de densité relative de pétrole brut ;
la détermination d'un poids moléculaire effectif du pétrole brut sur la base de la valeur de densité relative, dans lequel le poids moléculaire effectif ($MW_{EFF}$) est déterminé en utilisant :

$$MW_{EFF} = k_6 + k_7\gamma_{API} - (k_8 - k_9\gamma_{API})ln(\gamma_{API}),$$

où $\gamma_{API}$ est la valeur de gravité API pour le pétrole brut, et $k_6$ à $k_9$ sont des constantes prédéterminées ;
le calcul d'une valeur de viscosité de pétrole brut en fonction du poids moléculaire effectif ; et
le stockage ou l'affichage de la valeur de viscosité de pétrole brut.

**2.** Procédé mis en œuvre par ordinateur selon la revendication 1, dans lequel la détermination du poids moléculaire effectif comprend l'application d'un ajustement de caractérisation de Watson, éventuellement dans lequel l'ajustement de caractérisation de Watson est représenté par :

$$\Delta MW_{EFF} = k_{10}K_w - k_{11},$$

où $\Delta MW_{EFF}$ est un changement du poids moléculaire effectif, $K_w$ est un facteur de caractérisation de Watson et $k_{10}$ et $k_{11}$ sont des constantes prédéterminées.

**3.** Procédé mis en œuvre par ordinateur selon l'une quelconque des revendications 1 ou 2, dans lequel la détermination du poids moléculaire effectif comprend l'application d'un ajustement de teneur en gaz, dans lequel le poids moléculaire effectif avec un ajustement de teneur en gaz ($MW_{EFF,GCA}$) est représenté par :

$$MW_{EFF,GCA} = MW_{EFF} \times \frac{\rho_0 + nR_s\rho_{air}\gamma_g}{\rho_0 + R_s\rho_{air}\frac{MW_{EFF}}{MW_{air}}},$$

où $MW_{EFF}$ est une valeur de poids moléculaire effectif basée sur la densité relative du pétrole brut obtenue, $\rho_o$ est une valeur de densité du pétrole brut, $n$ est un facteur de non-idéalité, $R_s$ est un rapport gaz/pétrole à la pression de point de bulle, $\rho_{air}$ est une valeur de densité d'air, $\gamma_g$ est une valeur de densité de gaz, et $MW_{air}$ est une valeur de poids moléculaire d'air.

**4.** Procédé mis en œuvre par ordinateur selon l'une quelconque des revendications 1 ou 2, dans lequel la détermination du poids moléculaire effectif comprend l'application d'un ajustement de teneur en gaz et d'un ajustement de pression relative, dans lequel l'ajustement de pression relative est basé sur une comparaison d'une pression mesurée du pétrole brut avec une pression de point de bulle du pétrole brut, et dans lequel la valeur de viscosité de pétrole brut ($\mu$) est calculée comme suit :

$$\mu = \mu_{ob}exp[k_{12} \times (P - P_b)/\gamma_{API}],$$

où $\mu_{ob}$ est une valeur de viscosité de pétrole brut correspondant à un poids moléculaire effectif avec ajustement de teneur en gaz pour un rapport gaz/pétrole à la pression de point de bulle, calculée à l'aide des équations suivantes :

$$log[log(\mu_0 + 1)] = A - B log(T),$$

$$A = k_1(1 - exp(-k_2 MW) + k_3 MW,$$

$$B = k_4(1 - exp(-k_5 MW),$$

où MW est le poids moléculaire effectif avec ajustement de teneur en gaz ($MW_{EFF,GCA}$), $k_1$ à $k_4$ et $k_{12}$ sont une constante prédéterminée, $P$ est une pression mesurée du pétrole brut, $P_b$ est une pression de point de bulle du pétrole brut et $\gamma_{API}$ est une valeur de gravité API pour le pétrole brut.

5. Procédé mis en œuvre par ordinateur selon l'une quelconque des revendications 1 ou 2, dans lequel l'obtention de la valeur de densité relative comprend la collecte de mesures à partir de capteurs dans un trou de forage, et la dérivation d'une valeur de gravité API pour le pétrole brut sur la base des mesures collectées.

6. Procédé mis en œuvre par ordinateur selon l'une quelconque des revendications 1 ou 2, comprenant en outre :

le fait de déterminer si le pétrole brut est saturé, sur la base des mesures de température, de pression et de rapport pétrole/gaz, et l'application d'un ajustement de teneur en gaz au poids moléculaire effectif du pétrole brut en réponse au fait de déterminer que le pétrole brut est saturé, dans lequel le poids moléculaire effectif avec ajustement de teneur en gaz ($MW_{EFF,GCA}$) est représenté par :

$$MW_{EFF,GCA} = MW_{EFF} \times \frac{\rho_0 + nR_s\rho_{air}\gamma_g}{\rho_0 + R_s\rho_{air}\frac{MW_{EFF}}{MW_{air}}},$$

où $MW_{EFF}$ est une valeur de poids moléculaire effectif basée sur la densité relative obtenue du pétrole brut, $\rho_o$ est une valeur de densité du pétrole brut, $n$ est un facteur de non-idéalité, $R_s$ est un rapport gaz/pétrole à la pression de point de bulle, $\rho_{air}$ est une valeur de densité d'air, $\gamma_g$ est une valeur de densité de gaz et $MW_{air}$ est une valeur de poids moléculaire d'air ; et

le fait de déterminer si le pétrole brut est sous-saturé, sur la base des mesures de température, de pression et de rapport pétrole/gaz, et l'application d'un ajustement de teneur en gaz et d'un ajustement de pression relative au poids moléculaire effectif du pétrole brut en réponse à la détermination que le pétrole brut est sous-saturé, dans lequel l'ajustement de pression relative est basé sur une comparaison d'une pression mesurée du pétrole brut avec une pression de point de bulle du pétrole brut, et dans lequel la valeur de viscosité de pétrole brut ($\mu$) est calculée comme suit :

$$\mu = \mu_{ob} exp[k_{12} \times (P - P_b)/\gamma_{API}],$$

où $\mu_{ob}$ est une valeur de viscosité de pétrole brut correspondant à un poids moléculaire effectif avec ajustement de teneur en gaz pour un rapport gaz/pétrole à la pression de point de bulle, calculée à l'aide des équations suivantes :

$$log[log(\mu_0 + 1)] = A - B log(T),$$

$$A = k_1(1 - exp(-k_2 MW) + k_3 MW,$$

$$B = k_4(1 - exp(-k_5 MW),$$

où MW est le poids moléculaire effectif avec ajustement de teneur en gaz ($MW_{EFF,GCA}$), $k_{12}$ est une constante prédéterminée, P est une pression mesurée du pétrole brut, $P_b$ est une pression de point de bulle du pétrole brut et $\gamma_{API}$ est une valeur de gravité API pour le pétrole brut.

**7.** Procédé mis en œuvre par ordinateur selon l'une quelconque des revendications 1 ou 2, dans lequel la valeur de viscosité de pétrole brut est calculée par la mise à l'échelle d'une valeur de viscosité initiale ($\mu_0$) représentée par :

$$log[log(\mu_0 + 1)] = A - Blog(T),$$

où A = $k_1(1 - exp(-k_2 MW) + k_3 MW$ et B = $k_4(1 - exp(-k_5 MW))$ et où $MW$ est un poids moléculaire de pétrole brut, et $k_1$ à $k_5$ sont des constantes prédéterminées.

**8.** Système comprenant :

une unité de mémoire qui stocke une valeur de gravité API en tant que valeur de densité relative de pétrole brut ; et au moins une unité de traitement qui détermine un poids moléculaire effectif de pétrole brut sur la base de la valeur de densité relative, et calcule une valeur de viscosité de pétrole brut en fonction du poids moléculaire effectif, dans lequel le poids moléculaire effectif ($MW_{EFF}$) est déterminé en utilisant :

$$MW_{EFF} = k_6 + k_7 \gamma_{API} - (k_8 - k_9 \gamma_{API})ln(\gamma_{API}),$$

où $MW_{EFF}$ est le poids moléculaire effectif, $\gamma_{API}$ est la valeur de gravité API stockée pour le pétrole brut, et $k_6$ à $k_9$ sont des constantes prédéterminées,

**9.** Système selon la revendication 8,
dans lequel l'au moins une unité de traitement détermine le poids moléculaire effectif par l'application d'un ajustement de caractérisation de Watson.

**10.** Système selon la revendication 8,
dans lequel l'au moins une unité de traitement détermine le poids moléculaire effectif par l'application d'au moins un ajustement de teneur en gaz et d'un ajustement de pression relative.

**11.** Système selon la revendication 10,
dans lequel l'au moins une unité de traitement calcule la valeur de viscosité de pétrole brute par l'un des éléments suivants :

la mise à l'échelle d'une valeur de viscosité de pétrole brut initiale correspondant à un poids moléculaire effectif avec ajustement de teneur en gaz pour un rapport gaz/pétrole à la pression de point de bulle ; et
la mise à l'échelle d'une valeur de viscosité de pétrole brut standard sur la base du poids moléculaire effectif, de la température de pétrole brut et de la pression de pétrole brut.

**12.** Système selon l'une quelconque des revendications 8 à 11, comprenant en outre des capteurs dans un trou de forage pour collecter des mesures de pétrole brut, dans lequel les mesures de pétrole brut sont utilisées pour dériver la valeur de densité relative du pétrole brut.

**13.** Système selon l'une quelconque des revendications 8 à 11, dans lequel l'au moins une unité de traitement effectue une simulation de mouvement de fluide sur la base au moins en partie de la valeur de viscosité de pétrole brut calculée ; ou dans lequel l'au moins une unité de traitement dirige les opérations d'une unité de complétion de puits ou d'une unité de production sur la base au moins en partie de la valeur de viscosité de pétrole brut calculée.

FIG. 1

**FIG. 2A**

**FIG. 2B**

**FIG. 2C**

FIG. 3

FIG. 4

FIG. 5A

FIG. 5B

FIG. 6A

FIG. 6B

**FIG. 7A**

**FIG. 7B**

**FIG. 8A**

**FIG. 8B**

FIG. 9A

FIG. 9B

FIG. 10A

FIG. 10B

**FIG. 10C**

**FIG. 11**

302 — OBTAIN RELATIVE DENSITY VALUE OF CRUDE OIL

300 →

304 — DETERMINE $MW_{EFF}$

306 — CALCULATE VISCOSITY OF THE CRUDE OIL AS A FUNCTION OF $MW_{EFF}$

**FIG. 12**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 3720096 A

**Non-patent literature cited in the description**

- **H.P. ROENNINGSEN.** Prediction of viscosity and surface tension of North Sea petroleum fluids by using the average molecular weight. *ENERGY&FUELS,* September 1993, vol. 7 (5

- **YARRANTON et al.** Cold, Hot, or Dilute: Modeling the Viscosity of Heavy Oil for In Situ Processes. *GeoConvention,* 2013
- **BERGMAN et al.** A Consistent and Accurate Deal-Oil-Viscosity Method. *SPE Reservoir Evaluation & Engineering,* 2009, vol. 12 (6), 815-840